# EUROPEAN PATENT SPECIFICATION

(11) **EP 2 341 822 B1**
(45) Date of publication and mention of the grant of the patent: **11.06.2014**
(21) Application number: 09761793.0
(22) Date of filing: 12.06.2009
(51) Int. Cl.: A61B 5/00, A61B 5/103

(54) **ILLUMINATION APPARATUS FOR USE IN EXAMINING A BODY OF LIVING TISSUES**
BELEUCHTUNGSGERÄT ZUR VERWENDUNG BEI DER UNTERSUCHUNG EINES KÖRPERS AUS LEBENDEM GEWEBE
APPAREIL D'ÉCLAIRAGE POUR UNE UTILISATION DANS L'EXAMEN D'UNE MASSE DE TISSUS VIVANTS

(30) Priority: 13.06.2008 GB 0810890
(43) Date of publication of application: 13.07.2011
(73) Proprietor: PWB Health Limited, Dumbarton G82 3PW (GB)
(72) Inventor: DEVLIN, Ken, Dumbarton G82 3PW (GB); MCDAID, Andy, Ayrshire PA17 5DA (GB); WHITEHILL, Craig, Glasgow G42 8PT (GB); SAYER, Robin, Glasgow G14 9HE (GB); RUTHERFORD, David, Ardrossan KA22 8AX (GB)
(74) Representative: Jenkins, Peter David
(86) International application number: PCT/EP2009/057305
(87) International publication number: WO 2009/150231

(56) References cited:
- WO-A-2004/107977
- WO-A-2005/107577
- WO-A-2007/102036
- US-A1- 2005 213 317

## Description

The present invention relates to an illumination apparatus for use in examining a body of living tissues, in particular the female breast or male testicular tissues.

Breast cancer is the principal health concern of women and is the commonest cause of death in women under 50 years of age. In the UK there are 41,000 new cases per year and the trend is upwards. There are 13,000 deaths per year from breast cancer. The 5 year survival is 73%. This means 1 in 4 women die within 5 years of diagnosis. The appropriate management of patients with early disease reduces mortality. Presently 15% of women present with advanced disease. Earlier detection should also reduce the number of mastectomies in favour of breast conserving surgery. Similar problems concern testicular cancer in men.

The present applicant is the successor to the applicant of GB 2375672A, WO 03/077744 and WO2005107577, which are directed to an easy to use, light-based instrument, which when employed together with breast self examination is designed to indicate the need for women to consult a GP for advice and possible referral to a hospital setting. The device emits from a light source visible radiation that is sufficiently intense to be transmitted through the tissues of the user and the tissues on the other side of the tissues from the side against which the device is disposed can be viewed by the user. The instrument is generally used in a darkened room so that variations in brightness on the surface of a breast through which light is transmitted may be observed. Any shadows or other particular variations in the transmitted radiation can indicate the possibility of medical problems. The device assists women with the disease to present at an early stage for diagnosis and treatment□. The prior device is small, safe, simple to use and is non-diagnostic. It involves no ionising radiation, no UV radiation, or breast compression.

However, there is a need further to develop this device to ensure that it is easy to use and reassuring to the lay user.

WO-A-2007-102036 relates to an electro-optical device for screening of skin diseases.

This present invention therefore aims to provide improvements to the illumination instruments for examining a body of living tissues described in the above-mentioned earlier patent applications, in particular to provide reliable and user-friendly instruments.

Accordingly, in a first aspect the present invention provides an illumination apparatus for use in examining a body of living tissues, the apparatus comprising a head for contacting the body of living tissues, a light source mounted in the head for illuminating the tissues, and an annular switch disposed around the light source, the annular switch being adapted automatically to permit illumination of the light source at an examination intensity when the head contacts the body at least around an annular contact area on the head corresponding to the annular switch.

There is also disclosed an illumination apparatus for use in examining a body of living tissues, the apparatus comprising a head for contacting the body of living tissues, a light source mounted in the head for illuminating the tissues, the light source comprising a plurality of light emitting diodes, a power circuit for providing electrical power to the light emitting diodes and a switch for switching the electrical power to the light emitting diodes to modify the illumination of the light source from a relatively low level intensity standby mode to a relatively high level intensity examination mode, the power circuit providing pulse width modulated voltage to the light emitting diodes at least in the standby mode.

The use of pulse width modulated (PWM) voltage in the low power standby mode can fool the eye into seeing an equal brightness for the LEDs at low brightness levels for the LED output. In the high power examination mode, preferably a constant voltage is supplied to the LEDs, although optionally a PWM voltage may be employed to drive the LEDs in the high power examination mode as well as in the in the low power standby mode.

There is further disclosed an illumination apparatus for use in examining a body of living tissues, the apparatus comprising a head for contacting the body of living tissues, a light source mounted in the head for illuminating the tissues, a switch adapted automatically to permit illumination of the light source at an examination intensity when the head contacts the body in a predetermined manner, a clock for measuring time, a time recorder for recording elapsed time, as measured by the clock, since the switch was last activated and the light source was last illuminated for a predetermined minimum period, and a control circuit adapted to reset the time recorder to record a zero elapsed time, the control circuit being operable after the switch has been activated and the light source has been illuminated for the predetermined minimum period.

There is further disclosed an illumination apparatus for use in examining a body of living tissues, the apparatus comprising a head for contacting the body of living tissues, a light source mounted in the head for illuminating the tissues, and a power circuit for providing electrical power to the light source in an examination mode, wherein the power circuit is adapted to provide a plurality of illumination levels in the examination mode.

There is further disclosed an illumination apparatus for use in examining a body of living tissues, the apparatus comprising an elongate housing having a body portion and a head, at one end of the body portion, for contacting the body of living tissues, a light source mounted in the head for illuminating the tissues, and a heat sink member within the elongate housing, the heat sink member extending from adjacent to the light source into the body portion and along a majority of the length thereof.

Embodiments of the present invention will now be described by way of example only with reference to the accompanying drawings, in which:-
Figure 1 is a perspective view of an illumination apparatus for use in examining a body of living tissues, such as the female breast, in accordance with a first embodiment of the present invention;
Figure 2 is an exploded perspective view of the apparatus of Figure 1;
Figure 3 is a perspective partly cut-away view of a head portion of the apparatus of Figure 1;
Figures 4a and 4b are schematic views of the apparatus of Figure 1 when used to examine the female breast;
Figure 5 is a plan view of the display of the apparatus of Figure 1; and
Figure 6 shows schematically a control system for the apparatus of Figure 1.

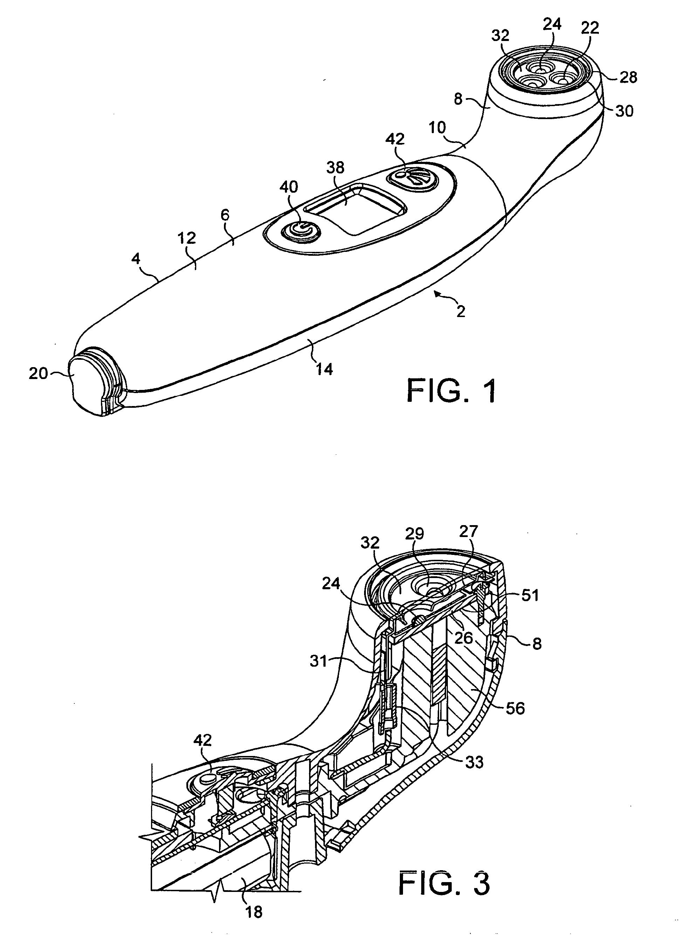

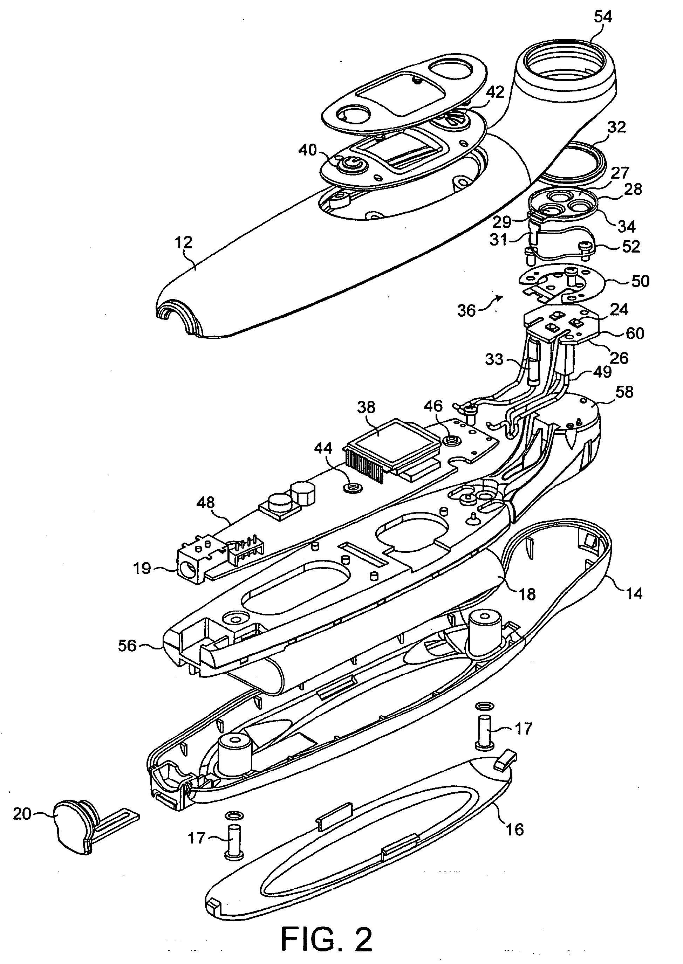

Referring to Figures 1, 2 and 3 of the accompanying drawings, there is shown a hand-held illumination apparatus 2 for use in examining a body of living tissues, such as the female breast, in accordance with an embodiment of the present invention. The apparatus 2 comprises an elongate housing 4 having a body portion 6 and a head 8 disposed at one end 10 of the body portion 6, the head 8 being shaped and configured for contacting the body of living tissues.

The housing 4 comprises an upper part 12 and a lower part 14 that are fitted together. The lower part 14 includes a removable cover 16 for the screws 17 that hold the upper part 12 and lower part 14 together. A power source, such as a rechargeable battery 18, is located within the housing 4, and the housing 4 includes a recharging socket for a jack plug 19 temporarily covered by a cover 20. The rechargeable battery 18 can be removed (as required by the WEEE directive) but can only be removed by unscrewing upper part 12 and lower part 14, to permit battery removal for repair or proper disposal at the end of the life of the apparatus 2.

A light source 22 is mounted in the head 8 for illuminating the tissues. The light source 22 comprises a plurality (in the illustrated embodiment three) of light emitting diodes 24 (LEDs) arranged in a triangular array. Each light emitting diode 24 has an emission wavelength in a red-orange region of the visible spectrum, and preferably each light emitting diode 24 has an emission wavelength within the range of from 613.5 to 620.5 Nanometers.

The LED wavelength is carefully chosen to be in the wavelength range that is absorbed by the haemoglobin in the blood. This will show up as dark regions where any angiogenesis is taking place on illumination of the breast tissue using the illumination apparatus 2 as described herein. Both red-orange and red light wavelength emitting diodes 24 (having a total emission wavelength range of from 613.5 to 640.5 Nanometers) will perform this function. However, the red-orange LED, being at a lower wavelength than red, stimulates the eye more (for a fixed light power). The red-orange was therefore chosen as the most preferred wavelength since it results in a better contrast.

The plurality of light emitting diodes 24 is mounted on a printed circuit board 26, typically composed of an epoxy resin fibre reinforced laminate having a copper electroconductive layer, the lower surface of the printed circuit board 26 being bonded for thermal contact to a metal, e.g. aluminium, substrate which is electrically isolated from the circuitry on the printed circuit board 26.

An annular switch 28 is disposed circumferentially around the light source 22. The annular switch 28 is adapted automatically to permit illumination of the light source at relatively high examination intensity when the head 8 contacts the body of tissues at least circumferentially around an annular contact area 30 on the head 8 corresponding to the annular switch 28. A window 32 covers the light source 22 and the annular switch 28 is covered by the window 32. The annular switch 28 comprises an electrically conductive, preferably metal, ring 34 that is part of a capacitive switch mechanism 36. The annular switch 28 has a central disc 27 with holes 29 therein for receiving a respective light emitting diode 24. The upper surface of the disc 27 acts as a reflector for light emitted from the light emitting diodes 24. An integral downward leg 31 of the annular switch 28 engages with an electrical crimp 33 and is electrically connected thereby to circuitry, as described further below. The annular switch 28 switches the illumination of the light source 22 from a relatively low level intensity standby mode to a relatively high level intensity examination mode after a predetermined delay of time after the head 8 contacts the body at least around the annular contact area 30 on the head 8 corresponding to the annular switch 28.

A display 38 is provided on the upper part 12 for displaying a variety of different parameters and functions. Adjacent to the display 38 are a power button 40 and an illumination intensity button 42, both buttons 40, 42 being manually operable. The display 38 and the switch mechanisms 44, 46 under the buttons 40, 42 are mounted on a main printed circuit board 48.

The main printed circuit board 48 is electrically connected by wiring 49 to the circuit board 26 on which the plurality of light emitting diodes 24 is mounted.

The circuit board 26 is covered by a LED mask 50 having holes corresponding to the LEDs. An annular compression spring 52 is located between the LED mask 50 and the annular switch 28 to bias the annular switch 28 upwardly against the window 32. The window 32 is in a hermetically sealing engagement with the periphery of a mounting hole 54 in the head 8, for example by the window 32 being ultrasonically welded into the head 8.

A heat sink member 56, preferably composed of a metal, is located within the elongate housing 4, the heat sink member 56 extending from adjacent to the light source 22 into the body portion 6 and along a majority of the length thereof, preferably along the entire length of the body portion 6. The upper face 58 of the heat sink member 56 is thermally coupled to the lower face 60 of the circuit board 26 on which the plurality of light emitting diodes 24 is mounted, optionally by way of a thermally conductive paste or coupling pad 51 therebetween.

The illumination apparatus 2 uses heat management techniques to provide a low temperature head containing the illuminated light emitting diodes which can maximise the time available for examination, can minimise current draw for a given light output and hence minimise the battery consumption, and can ensure that the light emitting diodes operate at maximum brightness (light emitting diode brightness tends to be reduced with a temperature rise at the LED junction). The heat sink is elongate and extends along the entire length of the hand-held illumination apparatus to draw heat from LED junction of the light emitting diodes and the elongate heat sink design removes the need for convective cooling and/or any requirement for vents in the housing around the light source. The light emitting diodes are mounted on a PCB which is thermally coupled to a metal e.g. aluminium support to minimise the thermal resistance between the light emitting diodes and the heat sink. The light emitting diodes are operated during the examination mode at the most efficient point on the light output/current curve to minimise the heat generated for a given light output.

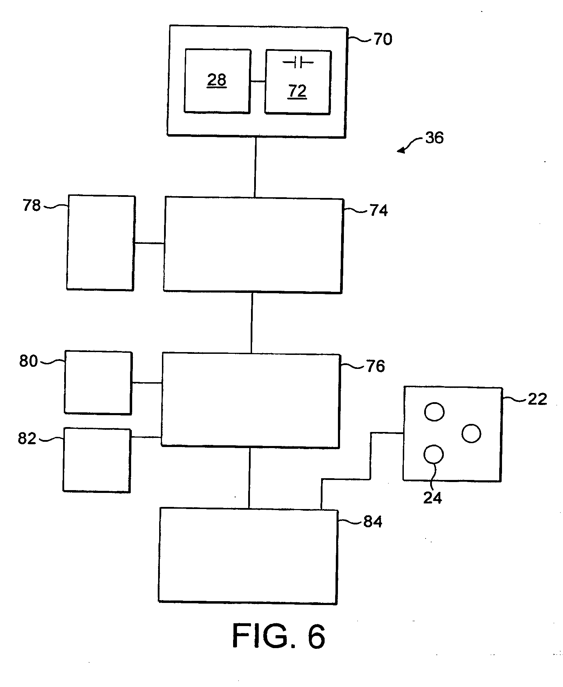

Referring to Figure 6, the capacitive switch mechanism 36 has a capacitive element 70, the capacitive element 70 including the electrically conductive annular switch 28 and, electrically connected thereto by the crimp 33, a capacitor 72 of predetermined or variable capacitance. A control mechanism 74 for the capacitive switch mechanism 36 is provided, the control mechanism 74 having a preset minimum capacitance threshold. When the capacitance of the capacitive element 70 is above the preset minimum capacitance threshold, by the head 8 contacting the body of living tissues at least around the annular contact area 30 on the head 8 corresponding to the annular switch 28, the capacitive switch 36 is switched on to permit an output from the control mechanism 74 to a controller or CPU 76.

A calibration system 78 for calibrating the preset minimum capacitance threshold for the capacitive element 70 is provided, which is triggered to operate when the illumination apparatus 2 is operated by a user.

The capacitive switch mechanism 36 is configured to normalise itself by a calibration protocol in order to suit the structural configuration and assembly, in particular the selected materials and their dielectric constants. The capacitive switch integrated circuit (IC) constituting the control mechanism 74 has a preset threshold level recorded in the circuitry. The capacitance of a combination of the set internal capacitor 72 along with that of the metal ring 28 of the annular switch (when isolated in the unit) is determined, and the threshold level is set to be above that capacitance. When the metal ring 28 is touched or is adjacent to the human body, for example by full skin coverage over the metal ring and/or window, the capacitance of the annular switch 28 changes, bringing the combined capacitance above the threshold level, and the capacitive switch integrated circuit (the control mechanism 74) correspondingly produces an output, thereby turning the capacitive switch mechanism 36 on.

Technically the capacitive switch integrated circuit is tuned to respond to a range of specific capacitances above the threshold - it detects capacitances within this range, and does not specifically detect skin.

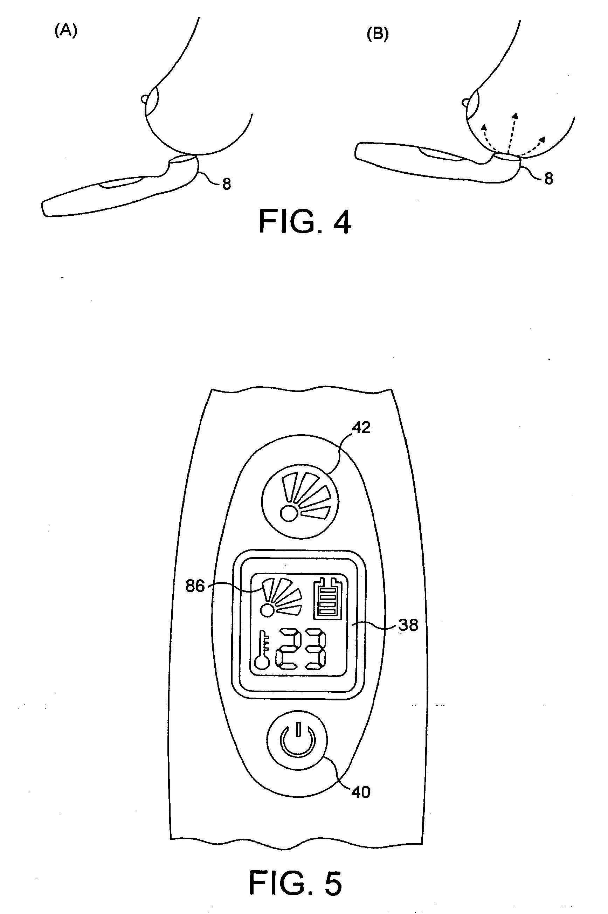

The metal ring of the annular switch 28 is in full surface annular contact with underside of the thin plastic window 32, which is typically about 1mm thick. The control mechanism 74, preferably comprised of a capacitive switch integrated circuit (IC), is calibrated by calibration system 78 to trigger capacitive switch mechanism 36 to be turned on with full skin contact of the head 8 onto the body of tissues. The metal ring fully encompasses the plural (e.g. 3) light emitting diode cluster constituting light source 22, thereby ensuring that when full skin to window contact occurs, as shown in Figure 4(b), this automatically triggers the capacitive switch mechanism 36 to illuminate the light emitting diodes 24 at their high intensity in the examination mode. Accordingly, in the high intensity examination mode, any light from the light emitting diodes 24 can only pass into the body, e.g. breast tissue, and does not escape. Conversely, as shown in Figure 4(a), in the absense of full skin contact of the head 8 onto the body of tissues, the capacitance of the capacitive element 70 is below the predetermined threshold, and power provided to the light emitting diodes 24 remains in the relatively low level intensity standby mode. In the examination mode, when the capacitive switch mechanism 36 has switched the light emitting diode cluster to full power, on a selected one of the plural (four, in the embodiment) modes, the LEDs are fully eye safe and will not cause any permanent eye damage. The main reasons to provide a switching to the examination mode only after full skin contact are to avoid dazzling the user, which could be a potential safety hazard in an otherwise dark environment, to ensure maintaining dark sensitivity for optimum visualisation of the illuminated tissue under low ambient light conditions, and also to avoid light spillage at point of contact with the body tissues and wastage of power.

The capacitive switch mechanism 36 and the light emitting diode cluster are capable of operating behind a hermetically sealed plastic enclosure formed by the window 32. This is advantageous for the illumination apparatus 2 because it is recommended that users apply a lubricant to their skin to allow for more comfortable movement of the window 32 against their skin during examination of the body tissue.

The internal set capacitance is set by calibrating the internal capacitor 72 when the illumination apparatus 2 is assembled, such that the capacitance of the entire illumination apparatus 2 and in particular of the heat-sink, the metal ring, and surrounding environment, is below the threshold level.

The internal capacitance, however, may be prone to slow drift over a prolonged period of time. Accordingly, on power-up, the system is adapted to calibrate itself such that any slow drift is compensated for, and that the capacitance of the isolated entire illumination apparatus 2 remains constant and below the threshold. Skin contact with the electrode constituted by the annular switch 28 or with the thin window 32 overlying the annular switch 28, results in a fast change of capacitance and this is what the IC (control mechanism 74) detects to trigger an output.

The trigger point is calibrated to operate effectively and reliably across a full range of users. The internal capacitance of the isolated entire illumination apparatus 2 is set to be below the threshold level; however the gain sensitivity of the IC (control mechanism 74) is calibrated such that all types of skin, independent of age, condition, environmental conditions, temperature, humidity such as perspiration, etc reliably triggers the desired output.

The light from the light emitting diodes 24 comes to full power over a period of 1 second to ensure that if someone activates the capacitive switch mechanism 36 by putting the head 8 right against skin in the region of their eye, then they have time to take action to move the illumination apparatus 2.

The capacitive switch mechanism 36 provides a number of technical benefits.

The capacitive switch mechanism 36 prevents user dazzle and allows the user's eye pupil to dilate and become sensitive to low light levels when the illumination apparatus 2 is used as recommended, under low ambient light conditions, to provide a high visual resolution and contrast of breast tissue. This is required because the amount of light that travels through the breast tissue is a fraction of that emitted by the illumination apparatus 2 so the user's eye must be sensitive to low light conditions. The user is advised to wait for a couple of minutes in a dark room before using the illumination apparatus 2. Note that a bright light at the operating wavelength is perfectly eye safe so this feature is not designed to solve a safety problem.

The capacitive switch mechanism 36 also prevents an accidental trigger, for instance by a finger touching the window 32, whilst reliably triggering when in position for a breast examination. Full surface contact around the metal ring of the annular switch 28 is required.

The capacitive switch mechanism 36 encourages a light seal against the breast surface, by only triggering when full contact is made with the window 32. A light seal ensures that the amount of light escaping is minimised. Light that escapes could dazzle the user and cause their pupils to contract, reducing sensitivity to low light conditions.

The capacitive switch mechanism 36 is also used to calculate number of days since last use, as described in detail below. Continuous activation of the capacitive switch mechanism 36 for more than 10 seconds is an indicator that the device has been used for its intended purpose and the days since last use indicator (visible on the display 38 such as an LCD screen) is reset to zero.

The capacitive switch mechanism 36 can also provide an intelligent auto-power-down function. The illumination apparatus 2 may be adapted only to power down (to save battery power) a particular time period, e.g. 1 minute, after the capacitive switch mechanism 36 is last deactivated, thus ensuring that power down doesn't happen during a breast examination.

The capacitive switch mechanism 36 provides a reflector surface for high-output light emitting diodes 24. The capacitive switch system mechanism 36 includes the central metal disc 27 which is in contact with the underside of the window 32. This disc 27 also acts as a reflector for the light emitting diodes 24 and ensures that light loss is minimised. The reflector 27 is only in capacitive contact with the window 32 around its periphery, and therefore any contact except around 360 degrees of the periphery will not trigger the switch 28.

The capacitive switch mechanism 36 can also be used to deactivate the display 38 (e.g. an LCD backlight) so that the user is not distracted by any peripheral illumination during the tissue examination

A clock 80 for measuring time is provided in combination with a time recorder 82 for recording elapsed time, as measured by the clock. The time recorder 82 is actuated to record elapsed time since the switch was last activated and the light source was last illuminated for a predetermined minimum period. The controller 76 is adapted to reset the time recorder 82 to record a zero elapsed time, the controller 76 being operable after the capacitive switch 36 has been activated and the light source 32 has been illuminated for the predetermined minimum period.

The time recorder 82 is configured whereby the elapsed time is measured in days. The clock 80 and time recorder 82 are configured so that on first activation of the capacitive switch 36 and illumination of the light source 22 for the predetermined minimum period, the elapsed time is recorded as zero.

The display 38 displays the currently recorded elapsed time, for example a period of 23 days, as shown in Figure 5.

This feature provides the benefit to the user of acting as a reminder for planning the subsequent tissue examination. The capacitive switch mechanism 36 is used to judge that illumination apparatus 2 has been 'used'. If the capacitive switch mechanism 36 is active for more than a predetermined minimum period, such as ten seconds, then the illumination apparatus 2 resets the parameter "number of days since last use" count to zero. The clock 80 provides a "Real-Time Clock (RTC)" functionality that may be accomplished with an inexpensive watch crystal to provide an accurate time base, and appropriate conventional software control, to calculate the time. The parameter "number of days since last use" is displayed on the display 38 in a two digit format. If number is greater than 99 then '99' is flashed on and off. The counting of elapsed days is activated after the first use of the device "out of the box", i.e. on first use the count will always be zero.

During manufacture and calibration of the apparatus, at completion of manufacture the unit is placed into an "engineering mode" which automatically resets the time recorder 82 to zero.

A power circuit 84 provides electrical power to the controller or CPU 76 and also to the light emitting diodes 24 of the light source 22. The capacitive switch 36 is arranged to switch the electrical power to the light emitting diodes 24 to modify the illumination of the light source from a relatively low level intensity standby mode to a relatively high level intensity examination mode. The power circuit 84 provides pulse width modulated voltage to the light emitting diodes 24 in the relatively low level intensity standby mode. The power circuit 84 provides constant voltage in the relatively high level intensity examination mode. Alternatively, the power circuit 84 may also provide pulse width modulated voltage in the examination mode.

The pulse width modulated voltage in the standby mode is arranged to provide each of the light emitting diodes 24 with substantially the same intensity of illumination in the standby mode. The power circuit 84 may be adapted to provide an independently controllable pulse width modulated voltage to each of the respective light emitting diodes 24.

The pulse width modulation (PWM) of the voltage supply for the light emitting diodes 24 is provided so that the illuminated light emitting diodes 24 all look the same at low brightness levels. The illumination apparatus 2 switches on to provide a very low brightness level in an initial standby mode and only comes on to a full power when the capacitive switch mechanism is triggered in an examination mode.

Typically, the voltage is modulated to provide a duty cycle of from 1% to 50%, more typically from 1 to 10%, most typically about 5%, in the low power standby mode and a duty cycle of from 50% to 100%, more typically from 90 to 100% in the high power examination mode (100% corresponding to a constant voltage).

The illustrated embodiment drives the high power examination mode at 100% duty cycle but in alternative embodiments the duty cycle may be less than 100% to provide a PWM voltage in both modes to adjust for brightness variations caused by LED tolerances in the examination mode as well as the standby mode.

The PWM control overcomes inherent differences in the brightness of light emitting diodes 24 at low power levels and ensures that light emitting diodes 24 all look like they are providing the same output. If this were not the case, then the unit might look faulty, even though this is not the case. In the examination mode at high power levels, the visually apparent (given that the human eye has a non linear response) illumination differences between different light emitting diodes 24 are much less variable.

The light output of the light emitting diodes 24 as perceived by the human eye is much more prone to variation at very low forward currents, such as those found when in the stand-by mode. PWM is used to periodically pulse the light emitting diodes 24 to a high level, where the human eye can't notice any non-uniformities. However they are only pulsed on for a very short time such that the human eye sees a duller light. With PWM, if the light emitting diode 24 is only on for 10% of the time, the human eye will perceive the light emitting diode 24 is only outputting 10% of the light power.

The present invention may additionally provide independent control of the brightness of the light emitting diodes 24, in addition to overcoming any standby brightness differences using the PWM. These differences arise because of the inherent manufacturing tolerances that affect LED brightness for a given drive current.

The power circuit 84 is also arranged to provide the electrical power to the light emitting diodes 24 having a current corresponding to a maximum light output of the light emitting diodes 24, the maximum being determined as the maximum value of a range of light outputs useful for the required tissue e.g. breast illumination (the light emitting diodes 24 may theoretically be powered to emit greater light output, but that may be too high for the desired tissue illumination to achieve good tissue contrast). The current corresponding to the maximum light output may be determined from a relationship, such as a graphical curve, between the light output and current for the light emitting diodes 24.

The power circuit 84 provides electrical power to the light source 22 in an examination mode, and is adapted to provide a plurality of illumination levels in the examination mode. The plurality of illumination levels provides a succession of visually distinguishable increasing illumination levels, the increase between the successive levels being non-linear. Preferably, the non-linear increase is discernable by the human eye, and typically the plurality of illumination levels comprise four levels that emit light at relative illumination levels of 25:40:65:100.

Button 42 provides a manually operable control which is operable to select a selected one of the plurality of illumination levels. The button 42 is operable to select the selected one of the plurality of illumination levels by successive pressing operations. The display 38 displays the currently selected illumination level by an icon or symbol 86, as shown in Figure 5.

Accordingly, the illumination levels are preset so that differences between the consecutive levels are discernable visually to the human eye and are mapped to a non-linear user eye response. Typically, the preset illumination levels provide a light output of 25K, 40K, 65K and 100K Lux. In the examination mode, the light emitting diodes 24 may be provided with a constant voltage or a pulse width modulated (PWM) voltage. The single button 42 provides for actuation of the levels, with LCD feedback via a brightness symbol 86 with 1 of 4 levels displayed on the display 38. This simplifies the operation of the device for use in a dark environment.

The selection of the plural power levels is based on research carried out on a broad range of women, for example different skin types, different breast sizes, different tissue types, different age groups, women with breast implants, etc., and has been optimised for 4 levels across a range of women. This provides the benefit of minimising the number of distinct illumination levels so that user interface is simple, and can be easily operated in the dark, whilst ensuring that range of brightness levels is enough to provide the user with an optimum view of internal breast structure with optimum contrast between light and dark. The brightness range can cover illumination requirements for all potential users and all areas on the breast. The highest brightness level is selected to avoid over-illumination, i.e. a device with even brighter LEDs could be provided but this would over-illuminate the breast and reduce the contrast between light and dark areas of the breast.

The LED brightness in each of the four (e.g. 25,40,65,100 Lux) full power examination mode levels is controlled by a predetermined resistor, R2, R3, R4, R5, that is switched into circuit in parallel with a resistor R1. R1 is the resistor that governs the brightness of the LED's in the low power setting standby mode. The first full power brightness level is therefore achieved by switching R1 and R2 in parallel, the second brightness level is achieved by switching R1 and R3 in parallel and so on for the third and fourth levels. The brightness levels required to meet the full range of variability in breast tissue effectively define the relative values of R2-R5 and hence the value of R1. In setting mode the LED's are driven by only having R1 in the circuit.

Because of the relative values of R1 compared to the R2-R5 range the LED's when driven by R1 at 100% duty cycle are far brighter than desired for the low power setting mode. In addition the brightness variation due to tolerances across the three LEDs at that power level can easily be perceived by the human eye. The solution therefore adopted in accordance with this aspect of the present invention is to pulse width modulate the LEDs on a low, e.g. 5%, duty cycle at the low power setting which pulls down the brightness to a level consistent with the requirements of the low power setting mode and where the human eye cannot readily perceive any difference in brightness due to tolerances across the three LEDs used.

The present invention provides a small, safe, low cost, battery powered device which indicates the possibility of abnormalities and/or disease, such as breast disease, and indicates the need for a user to go for medical advice and further investigation. The present invention describes a device for use by women in the home. It can also be used by surgical, medical or nursing professionals to assist diagnosis to confirm the presence of a mass within breast tissues. The device can be used in a technique of breast self examination which can reduce the number of women presenting to medical professionals with advanced cancer and can limit the percentage of mastectomies. It is likely that patients with locally advanced breast cancer might be monitored to demonstrate the efficacy of chemotherapy or angiogenesis inhibiting drugs on their tumour using the device of the present invention.

## Claims

1. An illumination apparatus (2) for use in examining a body of living tissues, the apparatus (2) comprising a head for contacting the body of living tissues, a light source (22) mounted in the head (8) for illuminating the tissues, and **characterised by** an annular switch (28) disposed around the light source (22), the annular switch (28) being adapted automatically to permit illumination of the light source (22) at an examination intensity when the head (8) contacts the body at least around an annular contact area on the head (8) corresponding to the annular switch (28).

2. An apparatus according to claim 1 further comprising a window (32) covering the light source (22) and wherein the annular switch (28) is covered by the window (32).

3. An apparatus according to claim 1 or 2 where the annular switch (28) comprises an electrically conductive ring (34) that is part of a capacitive switch mechanism (36).

4. An apparatus according to any one of claims l, 2 and 3 wherein the capacitive switch mechanism (36) has a capacitive element (70), the capacitive element (70) including the electrically conductive ring 934), and a control mechanism, the control mechanism having a preset minimum capacitance threshold, whereby when the capacitance of the capacitive element (70) is above the preset minimum capacitance threshold, by the head (8) contacting the body of living tissues at least around the annular contact area on the head (8) corresponding to the annular switch (28), the capacitive switch (36) is switched on.

5. An apparatus according to any foregoing claim wherein the capacitive element (70) further includes a capacitor (72) of predetermined or variable capacitance.

6. An apparatus according to claim 4 or claim 5 further comprising a calibration system (78) for calibrating the preset minimum capacitance threshold for the capacitive element (70).

7. An apparatus according to claim 6 wherein the calibration system (70) is triggered to operate when the illumination apparatus (2) is operated by a user.

8. An apparatus according to any foregoing claim wherein the light source (22) comprises a plurality of light emitting diodes (24).

9. An apparatus according to any foregoing claim wherein the annular switch (28) switches the illumination of the light source (22) from a relatively low level intensity standby mode to a relatively high level intensity examination mode.

10. An apparatus according to any foregoing claim wherein the annular switch (28) switches the illumination of the light source (22) to the examination intensity after a predetermined delay of time after the head (8) contacts the body at least around the annular contact area on the head (8) corresponding to the annular switch (28).

11. An apparatus according to any foregoing claim wherein the light source (22) comprises a plurality of light emitting diodes (24), and further comprising a power circuit (84) for providing electrical power to the light emitting diodes (24), the switch (28) being arranged to switch the electrical power to the light emitting diodes (24) to modify the illumination of the light source (22) from a relatively low level intensity standby mode to a relatively high level intensity examination mode, the power circuit (84) providing pulse width modulated voltage to the light emitting diodes (24) at least in the standby mode.

12. An apparatus according to claim 11 wherein the pulse width modulated voltage has a low duty cycle in the standby mode and the power circuit (84) provides constant voltage to the light emitting diodes (24) in the examination mode.

13. An apparatus according to claim 11 or claim 12 wherein the pulse width modulated voltage in the standby mode is arranged to provide each of the light emitting diodes (24) with substantially the same intensity of illumination in the standby mode.

14. An apparatus according to any one of claims 11 to 13 wherein the power circuit (84) is adapted to provide an independently controllable pulse width modulated voltage to each of the light emitting diodes (24).

15. An apparatus according to any foregoing claim further comprising a clock (80) for measuring time, a time recorder (82) for recording elapsed time, as measured by the clock (80), since the switch (28) was last activated and the light source (22) was last illuminated for a predetermined minimum period, and a control circuit (76) adapted to reset the time recorder (32) to record a zero elapsed time, the control circuit (76) being operable after the switch (28) has been activated and the light source (22) has been illuminated for the predetermined minimum period, optionally where the clock (80) and time recorder (82) are configured so that on first activation of the switch (28) and illumination of the light source (22) for the predetermined minimum period the elapsed time is recorded as zero.

## Patentansprüche

1. Ein Beleuchtungsgerät (2), das zur am lebenden Körper zur Gewebeuntersuchung verwendet wird, wobei das Gerät (2) einen Kopf zum Berühren des lebenden Körpergewebes sowie eine im Kopf (8) montierte Lichtquelle (22) zum Beleuchten des Gewebes umfasst und **dadurch gekennzeichnet ist, dass** die Lichtquelle (22) von einem ringförmigen Schalter (28) eingefasst ist, wobei der ringförmige Schalter (28) vorgesehen ist, um eine automatische Beleuchtung der Lichtquelle (22) mit einer Untersuchungstärke zu ermöglichen, wenn der Kopf (8) den Körper zumindest mit seinem kreisförmigen Kontaktbereich berührt, der mit dem ringförmigen Schalter (28) übereinstimmt.

2. Ein Anspruch 1 entsprechendes Gerät, das ferner ein Fenster (32) zum Abdecken der Lichtquelle (22) und des ringförmigen Schalters (28) umfasst.

3. Ein Anspruch 1 oder 2 entsprechendes Gerät, wobei der ringförmige Schalter (28) einen elektrisch leitenden Ring (34) umfasst, der Teil eines kapazitiven Schaltmechanismus (36) ist.

4. Ein einem der Ansprüche 1, 2 und 3 entsprechendes Gerät, wobei der kapazitive Schaltmechanismus (36) ein den elektrisch leitenden Ring (34) umfassendes, kapazitives Element (70) und einen Kontrollmechanismus mit einem voreingestellten Mindestkapazitätschwellenwert hat, sodass der kapazitive Schalter (36) eingeschaltet wird, wenn die Kapazität des kapazitiven Elements (70) den voreingestellten Mindestkapazitätschwellenwert überschreitet, sobald der Kopf (8) das lebende Körpergewebe zumindest mit seinem kreisförmigen Kontaktbereich berührt, der dem ringförmigen Schalter (28) entspricht.

5. Ein einem vorstehenden Anspruch entsprechendes Gerät, wobei das kapazitive Element (70) ferner einen Kondensator (72) mit vorbestimmter oder variabler Kapazität umfasst.

6. Ein Anspruch 4 oder Anspruch 5 entsprechendes Gerät, das ferner ein Kalibrierungssystem (78) umfasst, um den voreingestellten Mindestkapazitätschwellenwert für das kapazitive Element (70) zu kalibrieren.

7. Ein Anspruch 6 entsprechendes Gerät, wobei das Kalibrierungssystem (70) aktiviert wird, wenn ein Benutzer das Beleuchtungsgerät (2) in Betrieb setzt.

8. Ein einem vorstehenden Anspruch entsprechendes Gerät, wobei die Lichtquelle (22) aus einer Vielzahl von lichtemittierenden Dioden (24) besteht.

9. Ein einem vorstehenden Anspruch entsprechendes Gerät, wobei der ringförmige Schalter (28) die Beleuchtung der Lichtquelle (22) von einem Bereitschaftmodus mit relativ niedriger Lichtstärke auf einen Untersuchungsmodus mit relativ hoher Lichtstärke umschaltet.

10. Ein einem vorstehenden Anspruch entsprechendes Gerät, wobei der ringförmige Schalter (28) die Beleuchtung der Lichtquelle (22) nach einer vorbestimmten Zeitverzögerung, nachdem der Kopf (8) den Körper zumindest in dem seinem ringförmigen Schalter (28) entsprechenden kreisförmigen Kontaktbereich berührt hat, auf Untersuchungsstärke umschaltet.

11. Ein einem vorstehenden Anspruch entsprechendes Gerät, wobei die Lichtquelle (22) eine Vielzahl von lichtemittierenden Dioden (24) und ferner einen Stromkreis (84) zur Stromversorgung der lichtemittierenden Dioden (24) umfasst, wobei der Schalter (28) vorgesehen ist, die Stromversorgung der lichtemittierenden Dioden (24) einzuschalten, um die Beleuchtung der Lichtquelle (22) von einem Bereitschaftmodus mit relativ niedriger Lichtstärke auf einen Untersuchungsmodus mit relativ hoher Lichtstärke zu modifizieren, wobei der Stromkreis (84) den lichtemittierenden Dioden (24) zumindest im Bereitschaftsmodus eine pulsweitenmodulierte Spannung zuführt.

12. Ein Anspruch 11 entsprechendes Gerät, wobei die pulsweitenmodulierte Spannung im Bereitschaftsmodus eine geringe Einschaltdauer hat und der Stromkreis (84) den lichtemittierenden Dioden (24) im Untersuchungsmodus eine konstante Spannung zuführt.

13. Ein Anspruch 11 oder Anspruch 12 entsprechendes Gerät, wobei die pulsweitenmodulierte Spannung im Bereitschaftsmodus vorgesehen ist, um jeder der lichtemittierenden Dioden (24) wesentlich die gleiche Beleuchtungsstärke im Bereitschaftsmodus zuzuführen.

14. Ein einem der Ansprüche 11 bis 13 entsprechendes Gerät, wobei der Stromkreis (84) vorgesehen ist, jeder der lichtemittierenden Dioden (24) eine unabhängig regelbare pulsweitenmodulierte Spannung zuzuführen.

15. Ein einem vorstehenden entsprechendes Gerät, das ferner Folgendes umfasst: eine Uhr (80) zur Zeitmessung; einen Zeitrekorder (82) zum Erfassen der abgelaufenen Zeit, die von der Uhr (80) gemessen wurde, seitdem der Schalter (28) zum letzten Mal betätigt wurde und die Lichtquelle (22) zum letzten Mal für eine vorbestimmte Mindestdauer aufleuchtete; sowie einen Regelkreis (76), der zum Rücksetzen des Zeitrekorders (32) vorgesehen ist, um eine abgelaufene Zeit von Null zu erfassen, wobei der Regelkreis (76) betriebsbereit ist, nachdem der Schalter (28) betätigt worden ist und die Lichtquelle (22) für die vorbestimmte Mindestdauer aufgeleuchtet hat, bzw. wahlweise, wobei die Uhr (80) und der Zeitrekorder (82) derartig konfiguriert sind, um eine abgelaufene Zeit von Null zu erfassen, wenn der Schalter (28) zum ersten Mal betätigt worden ist und die Lichtquelle (22) für die vorbestimmte Mindestdauer aufgeleuchtet hat.

## Revendications

1. Un appareil d'éclairage (2) pour une utilisation dans l'examen d'une masse de tissus vivants, l'appareil (2) comprenant une tête destinée à entrer en contact avec la masse de tissus vivants, une source lumineuse (22) montée dans la tête (8) pour éclairer les tissus, et **caractérisé par** un commutateur annulaire (28) disposé autour de la source lumineuse (22), le commutateur annulaire (28) étant adapté pour automatiquement permettre l'éclairage de la source lumineuse (22) à une intensité d'examen lorsque la tête (8) entre en contact avec la masse, au moins autour d'une zone de contact annulaire sur la tête (8) correspondant au commutateur annulaire (28).

2. Un appareil selon la revendication 1 comprenant en outre une fenêtre (32) qui couvre la source lumineuse (22) et dans lequel le commutateur annulaire (28) est couvert par la fenêtre (32).

3. Un appareil selon les revendications 1 ou 2 dans lequel le commutateur annulaire (28) comprend un anneau électriquement conducteur (34) qui fait partie d'un mécanisme de commutateur capacitif (36).

4. Un appareil selon l'une quelconque des revendications 1, 2, et 3 dans lequel le mécanisme de commutateur capacitif (36) possède un élément capacitif (70), l'élément capacitif (70) incluant l'anneau électriquement conducteur (34), et un mécanisme de contrôle, le mécanisme de contrôle possédant un seuil de capacité minimum prédéterminé, par lequel lorsque la capacité de l'élément capacitif (70) est au-dessus du seuil de capacité minimum prédéterminé, par la tête (8) entrant en contact avec la masse de tissus vivants au moins autour de la zone de contact annulaire sur la tête (8) correspondant au commutateur annulaire (28), le commutateur capacitif (36) est activé.

5. Un appareil selon l'une quelconque des revendications précédentes dans lequel l'élément capacitif (70) comprend en outre un condensateur (72) de capacité prédéterminée ou variable.

6. Un appareil selon la revendication 4 ou la revendication 5 comprenant en outre un système de calibrage (78) pour calibrer le seuil de capacité minimum prédéterminé pour l'élément capacitif (70).

7. Un appareil selon la revendication 6 dans lequel le système de calibrage (70) est déclenché pour fonctionner lorsque l'appareil d'éclairage (2) est actionné par un utilisateur.

8. Un appareil selon l'une quelconque des revendications précédentes dans lequel la source lumineuse (22) comprend une pluralité de diodes électroluminescentes (24).

9. Un appareil selon l'une quelconque des revendications précédentes dans lequel le commutateur annulaire (28) active l'éclairage de la source lumineuse (22) d'un mode de veille d'une intensité de niveau relativement faible jusqu'à un mode d'examen d'une intensité de niveau relativement élevé.

10. Un appareil selon l'une quelconque des revendications précédentes dans lequel le commutateur annulaire (28) active l'éclairage de la source lumineuse (22) à l'intensité d'examen après un laps de temps prédéterminé après que la tête (8) entre en contact avec la masse, au moins autour d'une zone de contact annulaire sur la tête (8) correspondant au commutateur annulaire (28).

11. Un appareil selon l'une quelconque des revendications précédentes dans lequel la source lumineuse (22) comprend une pluralité de diodes électroluminescentes (24), et comprend en outre un circuit de puissance (84) pour fournir une puissance électrique aux diodes électroluminescentes (24), le commutateur (28) étant agencé pour activer la puissance électrique aux diodes électroluminescentes (24) pour modifier l'éclairage de la source lumineuse (22) d'un mode de veille d'une intensité de niveau relativement faible jusqu'à un mode d'examen d'une intensité de niveau relativement élevé, le circuit de puissance (84) fournissant une tension modulée en largeur d'impulsion aux diodes électroluminescentes (24) au moins dans le mode de veille.

12. Un appareil selon la revendication 11 dans lequel la tension modulée en largeur d'impulsion possède un rapport cyclique faible dans le mode de veille et le circuit de puissance (84) fournit une tension constante aux diodes électroluminescentes (24) dans le mode d'examen.

13. Un appareil selon la revendication 11 ou la revendication 12 dans lequel la tension modulée en largeur d'impulsion dans le mode de veille est agencée pour fournir à chacune des diodes électroluminescentes (24) substantiellement la même intensité d'éclairage dans le mode de veille.

14. Un appareil selon l'une quelconque des revendications de 11 à 13 dans lequel le circuit de puissance (84) est adapté pour fournir une tension modulée en largeur d'impulsion contrôlable indépendamment à chacune des diodes électroluminescentes (24).

15. Un appareil selon l'une quelconque des revendications précédentes comprenant en outre une horloge (80) pour mesurer le temps, un enregistreur de temps (82) pour enregistrer le temps écoulé, tel que mesuré par l'horloge (80), depuis la dernière activation du commutateur (28) et le dernier éclairage de la source lumineuse (22) pendant une période minimum prédéterminée, et un circuit de contrôle (76) adapté pour remettre à zéro l'enregistreur de temps (32) pour enregistrer un temps écoulé zéro, le circuit de contrôle (76) étant opérable après l'activation du commutateur (28) et l'éclairage de la source lumineuse (22) pendant la période minimum prédéterminée, optionnellement dans lequel l'horloge (80) et l'enregistreur de temps (82) sont configurés de manière à ce que lors de la première activation du commutateur (28) et l'éclairage de la source lumineuse (22) pendant la période minimum prédéterminée le temps écoulé est enregistré comme zéro.
